# EUROPÄISCHE PATENTANMELDUNG

(11) **EP 3 528 257 A1**
(43) Veröffentlichungstag der Anmeldung: **21.08.2019**
(21) Anmeldenummer: 19157294.0
(22) Anmeldetag: 14.02.2019
(51) Int. Cl.: G16H 10/65, G06K 19/06

(54) **COMPUTERPROGRAMM, SYSTEM UND VERFAHREN ZUM VERWALTEN VON AUF EINEN PATIENTEN BEZOGENEN MEDIZINDATEN**

(30) Priorität: 14.02.2018 DE 102018103278
(71) Anmelder: Dugas, Martin, 48163 Münster (DE)
(72) Erfinder: Dugas, Martin, 48163 Münster (DE)
(74) Vertreter: Patentanwälte Geyer, Fehners & Partner mbB

(57) **Zusammenfassung**

Es wird beschrieben ein Verfahren zum Verwalten von auf einen Patienten bezogenen Medizindaten (22), für die ein vom Patienten zu besitzender Datenspeicher (6) vorgesehen wird, wobei im Datenspeicher (6) eine Datenbank (20) für die Medizindaten (22) vorgesehen ist, die Medizindaten (22) einem Gesundheitsdienstleister zur Verfügung gestellt werden, wobei ein Barcode (36) Verwendung findet, wobei der Datenspeicher (6) in ein Mobiltelefon (2) oder Tablet integriert ist, das einen Bildschirm (8) und eine Eingabevorrichtung (10) aufweist, und die Medizindaten (22) an den Gesundheitsdienstleister übertragen werden, indem der Patient mittels Bildschirm (8) und Eingabevorrichtung (10) aus der Datenbank (20) zu übertragende Medizindaten (22) auswählt, das Mobiltelefon (2) oder Tablet die zu übertragenden Medizindaten (22) in den Barcode (36) kodiert und diesen auf einem Medium zum Einlesen bereitstellt.

## Beschreibung

Medizindaten sind von zentraler Bedeutung für Prävention, Diagnostik und Therapie von Erkrankungen. Medizindaten sind patientenindividuelle Daten, die sich auf den Gesundheitszustand eines bestimmten Patienten beziehen. Beispiele sind Messdaten, beispielsweise Blutwerte, Blutdruckwerte oder Laborwerte, oder Befunde oder Diagnosen, wie sie beispielsweise in sogenannten Arztbriefen dokumentiert und mitgeteilt werden. Die Medizindaten sind auf einen bestimmten Patienten bezogen. Sie gehören diesem Patienten und sind durch die ärztliche Schweigepflicht sowie weitere Rechtsvorschriften geschützt.

Zugleich hat der Patient ein hohes Interesse daran, dass die Medizindaten in aktueller, korrekter und vollständiger Form dem jeweiligen Gesundheitsdienstleister, z.B. Ärzten oder anderen Mitarbeitern, zur Verfügung stehen. Die Erhebung von Medizindaten im Rahmen der Anamnese (v.a. Art und Verlauf der Symptome, Medikation, frühere Erkrankungen) ist von zentraler Bedeutung für das ärztliche Handeln.

Die medizinische Versorgung ist hochgradig spezialisiert, daher ist eine Vielzahl von unterschiedlichen Ärzten in die Versorgung eines Patienten involviert. Abgesehen von wenigen gesetzlich geregelten Situationen zur Weitergabe von Medizindaten (z.B. zu Abrechnungsdaten an die Kostenträger oder bei meldepflichtigen Erkrankungen nach dem Infektionsschutzgesetz) hat der Patient die Entscheidungshoheit, wem er welche seiner Medizindaten zur Verfügung stellt. Die Weitergabe dieser Daten außerhalb eines konkreten Behandlungsfalls muss durch den Patienten individuell autorisiert werden.

Durch die zunehmende Digitalisierung der Medizin werden immer mehr Medizindaten in digitaler Form erhoben und verarbeitet. Dies betrifft sowohl die von ärztlicher Seite erhobenen Daten, als auch die Daten, die der Patient selbst erhebt, insbesondere bei chronischen Erkrankungen. Typische Beispiele für die zweite Kategorie sind Patiententagebücher mit der Aufzeichnung von Symptomen (z.B. Schmerzen), Messungen (z.B. Blutdruck, Blutzucker) und Maßnahmen (z.B. Einnahme von Medikamenten).

In der Praxis führt dies zu separaten Datensammlungen für jeden Behandlungsfall, oftmals in getrennten Datenbanken. Es gibt eine Vielzahl von Softwaresystemen, die in der stationären und ambulanten Behandlung eingesetzt werden, die jedoch dem Patienten derzeit in der Regel keinen elektronischen Zugang zu seinen eigenen Daten bieten. Sie sind letztlich Insellösungen und Medizindaten sind derzeit hochgradig fragmentiert. Es stellt sich das grundsätzliche Problem, wie der Patient über die Weitergabe seiner Daten in elektronischer Form entscheiden soll, wenn er selbst in der Regel keinen Zugang zu diesen Daten hat.

Aufgrund der hohen Anzahl von Patienten im Verhältnis zu behandelnden Ärzten ist das Zeitkontingent für das ärztliche Patientengespräch begrenzt und ein effizienter Datenaustausch zwischen Arzt und Patient wäre wünschenswert. Dies betrifft sowohl die ärztliche Anamnese (zu Symptomen, Medikation, früheren Erkrankungen) als auch die Information des Patienten über aktuelle Befunde (z.B. Laborwerte, bildgebende Verfahren) und ärztliche Behandlungsempfehlungen (z.B. Medikation). Wichtig ist, dass Patientendaten im Rahmen des ärztlichen Patientengesprächs möglichst vollständig, korrekt, nachvollziehbar und zeitsparend übermittelt werden. Beim Einsatz elektronischer Systeme ist zu beachten, dass diese für die Patienten einfach und zuverlässig bedienbar sein müssen (ein Großteil der Patienten ist durch die jeweilige Erkrankung oder altersbedingt in seinen Handlungsmöglichkeiten eingeschränkt) und zugleich eine sichere und datenschutzkonforme Datenverarbeitung ermöglichen.

In der ambulanten und stationären Patientenversorgung wird eine Vielzahl von Softwaresystemen eingesetzt. Im Bereich der niedergelassenen Ärzte gibt es alleine in Deutschland mehr als 100 verschiedene zugelassene Arztpraxisinformationssysteme (z.B. MEDISTAR, Turbomed, ALBIS der Firma CompuGroup). Auch im stationären Bereich gibt es eine größere Anzahl an Softwareprodukten (z.B. ORBIS der Firma Agfa, i.s.h.med. der Firma Cerner, medico der Firma Cerner). Für den Datenaustausch zwischen Ärzten werden in Deutschland u. a. xDT-Standards eingesetzt. Für den Datenaustausch zwischen IT-Systemen in Krankenhäusern werden HL7-Standards eingesetzt. Diese Standards ermöglichen den Datenaustausch innerhalb einer Institution (z.B. zwischen dem Laborsystem und dem klinischen Arbeitsplatzsystem), aber in der Regel nicht über Institutionen hinweg.

Die EP 2998892 A1 sieht einen Datenspeicher vor, der sich im Besitz des Patienten befindet, nämlich in Form einer Gesundheitskarte, welche einen Datenspeicher enthält. Auf diesem Datenspeicher werden Medizindaten des Patienten gespeichert. Dies veranlasst der Arzt etc. Dazu wird der Datenspeicher in ein Lesegerät eingelegt, das mit einem Datenbanksystem des Arztes verbunden ist. Auf diese Weise können Daten in den Datenspeicher eingeschrieben bzw. aus diesem ausgelesen werden. Die Gesundheitsdaten sind dabei in einer Ausführungsform in XML-Format abgelegt, und jeder Datensatz kann in einem Datenfeld einen Barcode zur Identifikation des individuellen Eintrags enthalten. Dieser Barcode kann zum Zugriff auf den entsprechenden Datensatz der Medizindaten verwendet werden. Das Prinzip der EP 2998892 A1 erlaubt es einem Patienten, ihn betreffende Medizindaten zu sammeln. Da dieses Konzept jedoch von einem Versicherungsdienstleister ausgeht, der die Gesundheitskarte mit dem Datenspeicher zur Verfügung stellt, ist das Vorgehen nicht dahingehend ausgerichtet, dass der Patient die Hoheit über die Weitergabe seiner Medizindaten hätte. Vielmehr widmet sich die Patentveröffentlichung nur der Problematik, wie das Datenbanksystem des Gesundheitsdienstleisters geschützt werden kann. Ein Einfluss des Patienten auf die Aufnahme oder Weitergabe von Daten auf seinem Datenspeicher ist nicht gegeben, sobald er seinen Datenträger zur Verfügung gestellt oder in das Lesegerät eingelegt hat. Dieses Konzept ist somit unter Datenschutzgesichtspunkten nachteilig.

Barcodes sind im Gesundheitswesen auch im Einsatz, um Medikamente oder Medikationspläne zu bezeichnen.

Der Erfindung liegt die Aufgabe zugrunde, die Nachteile des Standes der Technik zu beheben und die Verwaltung von auf einen Patienten bezogenen Medizindaten in die Hoheit des Patienten zu stellen und dennoch einen unkomplizierten Austausch von Medizindaten zu ermöglichen, der sowohl auf Seiten des Patienten als auch auf Seiten des Gesundheitsdienstleisters keine besonderen technischen Anforderungen stellt.

Die Erfindung ist in den unabhängigen Ansprüchen definiert. Die abhängigen Ansprüche betreffen bevorzugte Weiterbildungen.

Die Erfindung kann als Computerprogramm zum Verwalten von auf einen Patienten bezogenen Medizindaten realisiert sein, insbesondere als Computerprogrammprodukt. Das Computerprogramm weist einen Programmcode auf, der zur Ausführung auf einem Prozessor eines Mobiltelefons oder Tablets ausgebildet ist. Das Mobiltelefon oder Tablet weist einen Bildschirm, eine Eingabevorrichtung und einen Datenspeicher auf. Die Eingabevorrichtung kann beispielsweise als Touch-Screen ausgebildet werden. Eine Tastatur ist gleichermaßen möglich. Das Computerprogramm verfügt über ein Datenbankmodul, das Medizindaten im Datenspeicher verwaltet. Weiter verfügt es über ein Ausgabemodul. Das Ausgabemodul liest auf Anforderung durch einen Benutzer aus dem Datenspeicher mehrere der Medizindaten aus und stellt auf dem Bildschirm eine Auswahlanzeige dar. Diese Auswahlanzeige zeigt mehrere der Medizindaten zur Auswahl an. Dabei kann natürlich auch nur eine Kurzinformation angezeigt werden, beispielsweise ein Kurztext und ein Datum, das sich auf die Medizindaten bezieht. Das Datum kann beispielsweise das Datum einer Laboruntersuchung, eines Befundes, einer Diagnose oder eines Arztbriefes sein. Die derart angezeigten Medizindaten sind über die Eingabevorrichtung auswählbar. Der Benutzer wählt nun diejenigen Medizindaten an, die er übertragen möchte. Daraufhin kodiert ein Kodierungsmodul die markierten und damit ausgewählten Medizindaten in einen Barcode. Ein Übertragungsmodul stellt den Barcode zum Einlesen durch einen Gesundheitsdienstleister bereit. Hierfür wird ein geeignetes Medium verwendet, beispielsweise der Anzeigebildschirm des Mobiltelefons oder Tablets. Durch den Barcode ist automatisch sichergestellt, dass die Übertragung nicht rückkanalfähig ist. Es gibt für andere IT-Systeme dann keinen Zugriff auf die Datenbank, sondern immer nur zu den Medizindaten, die im Barcode kodiert sind.

Ein System zum Verwalten von auf den Patienten bezogenen Medizindaten umfasst neben einer solchen Vorrichtung auch eine Datenbank für Gesundheitsdienstleister zur Verwaltung von Medizindaten. Die Datenbank verfügt über ein Ausgabemodul, das vom Gesundheitsdienstleiser ausgewählte, auf den Patienten bezogene Medizindaten in einem Barcode kodiert und diesen auf einem Medium ausgibt.

In einer Weiterbildung umfasst die Datenbank auch ein Eingabemodul, das einen dem Patienten bereitgestellten Barcode einliest, die darin kodierten Medizindaten dekodiert und in der Datenbank ablegt. Die Übertragung per Barcode ist der vorgesehene Standardübertragungsweg, da er äußerst datensicher ist und die geringsten Hardware-Anforderungen bei Sender und Empfänger stellt. Dies schließt natürlich nicht aus, dass zusätzlich weitere Übertragungswege angeboten werden, um Daten in die Datenbank auf dem Mobiltelefon/Tablet zu übernehmen oder daraus auszugeben. Es ist als Ergänzung möglich, dass unter Kontrolle des Patienten Daten über andere Kanäle, beispielsweise via Bluetooth oder IP-basierte Netzwerke übertragen werden. Hierbei ist zweckmäßigerweise die Kontrolle und Autorisierung durch den Inhaber des Mobiltelefons/Tablets, also durch den Patienten vorgesehen. Z.B. kann zum Auslösen/Freigeben der Übertragung ein Barcode erzeugt und verwendet werden.

Ein Verfahren zum Verwalten von auf den Patienten bezogenen Medizindaten sieht einen dem Patienten zugeordneten Datenspeicher vor. Im Datenspeicher ist eine Datenbank für die Medizindaten vorgesehen. Der Patient gibt ein Auslesen von Medizindaten frei, um im Datenspeicher abgelegte Medizindaten an einen Gesundheitsdienstleister zu übertragen. Der Datenspeicher ist in ein Mobiltelefon oder Tablet integriert, das einen Bildschirm oder eine Eingabevorrichtung aufweist. Der Lesezugriff erfolgt, indem der Patient mittels Bildschirm und Eingabevorrichtung aus den in der Datenbank abgelegten Medizindaten eine Auswahl trifft. Er wählt die zu übertragenden Medizindaten aus. Das Mobiltelefon oder Tablet kodiert die zu übertragenden Medizindaten in einen Barcode. Es stellt diesen Barcode auf einem Medium zum Einlesen durch den Gesundheitsdienstleister bereit. Durch den Einsatz des Barcodes ist automatisch sichergestellt, dass kein unerwünschter Rückkanal besteht. Der Patient kann damit sicherstellen, dass nur diejenigen Medizindaten an den Gesundheitsdienstleiser übertragen werden, die er zuvor ausgewählt hat.

Es ist deshalb auch vorgesehen ein Computerprogrammprodukt mit Programmcode zum Ausführen des genannten Verfahrens.

Der Auswahlschritt ist von entscheidender Bedeutung. Er hat eine Doppelfunktion. Zum einen stellt er zusammen mit der Bereitstellung des Barcodes das Einverständnis des Patienten dar, überhaupt Medizindaten zu übertragen. Zum anderen erlaubt er es dem Patienten, gezielt auszuwählen, welche Medizindaten er bereitstellen möchte. Die Zusammenfassung der Medizindaten in einen Barcode erfolgt auf Seiten des Patienten in dessen Mobiltelefon oder Tablet. Diese Zusammenfassung unterscheidet sich grundlegend vom bisherigen Einsatz eines Barcodes im Stand der Technik, der eine feststehende Information, beispielsweise einen Medikationsplan oder einen bestimmten Befund kodierte. Die freie Einflussnahme des Patienten auf die Medizindaten, welche im Barcode enthalten sind und die Erzeugung des Barcodes erst auf Anforderung durch den Patienten sichern in Kombination miteinander zum einen die Herrschaft des Patienten über seine Medizindaten und zum anderen eine komplikationslose und aufwandsgeringe Übertragung der Medizindaten.

Das Verfahren kann natürlich analog zum Computerprogramm, der Vorrichtung und dem System so weitergebildet werden, dass das Mobiltelefon oder Tablet auch eine Barcode-Leseeinrichtung aufweist. Ein Schreib-Zugriff auf die Datenbank wird individuell freigegeben, um bei einem Gesundheitsdienstleister erzeugte Medizindaten im Datenspeicher abzulegen. Dies geschieht dadurch, dass der Gesundheitsdienstleister die Medizindaten in einem Barcode kodiert und diesen auf einem Medium bereitstellt. Der Patient liest den Barcode mittels der Barcode-Leseeinrichtung ein. Das Mobiltelefon oder Tablet dekodiert die Medizindaten aus dem Barcode und legt sie in der Datenbank ab. Dieses Verfahren ist besonderes dann einfach anzuwenden, wenn der Gesundheitsdienstleister den Barcode auf gedruckte Unterlagen bereitstellt, die er dem Patienten ohnehin mitgibt, beispielsweise einen Arztbrief. Der Patient kann dann auf diese Weise sehr einfach die Medizindaten in seine Datenbank einpflegen.

Die Erfindung sieht somit Methoden vor, die den Austausch von Medizindaten zwischen Arzt und Patienten unabhängig vom verwendeten IT-System des Arztes effizient unterstützen. Der Datenaustausch basiert auf Barcodes. Diese haben sich als überraschend einfache Maßnahme erwiesen, wie der Patient die Aufnahme von Medizindaten in eine Datenbank, die von ihm selbst gehalten wird, und zugleich auch die Übertragung von Medizindaten an einen Gesundheitsdienstleister frei steuern kann. Durch die Erfindung wird ein wichtiger Beitrag zur optimalen Prävention, Diagnostik und Therapie in der Medizin geleistet, da auf einfache Weise sichergestellt werden kann, dass ein Gesundheitsdienstleister vom Patienten fehlerfreie Medizintaten in dem Umfang erhält, in dem er sie benötigt und der Patient bereit ist, sie ihm zur Verfügung zu stellen. Aufwendige Einverständniserklärungen sind nicht erforderlich, da die Bereitstellung durch den Patienten selbst erfolgt.

Der Einsatz von Barcodes vermeidet dabei das aufwändige Vergeben von Zugriffsrechten oder Datenbankabsicherungen, da Barcodes per se eine nicht rückkanalfähige Art der Datenübertragung sind. Stellt der Patient dem Gesundheitsdienstleister ausgewählte Medizindaten in Form eines Barcodes zur Verfügung, muss keine Vorsorge dafür getroffen werden, dass ein IT-System des Gesundheitsdienstleisters in einem vom Patienten nicht gewünschten Umfang Medizindaten aus der Datenbank, welche der Patient betreibt, einliest. Vielmehr ist der Patient alleiniger Herrscher sowohl über die Tatsache der Datenübertragung als auch über den Umfang der übertragenen Medizindaten. Hierfür ist es ganz wesentlich, dass der Patient die zu übertragenden Medizindaten aus seiner Datenbank auswählt und veranlasst, dass die ausgewählten Medizindaten in einen Barcode kodiert werden, welchen der Gesundheitsdienstleister dann einlesen kann. Dieses Vorgehen stellt sicher, dass nur vom Patienten gewünschte Medizindaten an den Gesundheitsdienstleister übertragen werden.

Da die Übertragung auf einem Barcode basiert, dessen Erzeugung der Patient selbst veranlasst hat, ist damit automatisch auch das Einverständnis des Patienten für die Weitergabe der Medizindaten an den Gesundheitsdienstleister sichergestellt. Zudem gibt der Barcode keinen Zugang zur Datenbank selbst, sondern nur zu den ausgewählten Medizindaten.

Die Erfindung ermöglicht auch in umgekehrter Richtung auf einfache Weise, dass der Patient die ihn betreffenden Medizindaten sammelt. Der Gesundheitsdienstleister stellt dazu in einer Weiterbildung einen Barcode zur Verfügung, welcher die Medizindaten kodiert. Beispielsweise kann der Barcode auf einen üblichen Arztbrief, mit dem der Gesundheitsdienstleister erhobene Patientendaten, Befunde oder Diagnosen mitteilt, aufgedruckt werden. Der Patient kann diesen Barcode einlesen und somit die darin kodierten Medizindaten seiner eigenen Datensammlung hinzufügen.

Die Datenbank, welche der Patient betreibt, ist in einem Mobiltelefon oder Tablet abgelegt. Auf diese Weise hat der Patient beim Gesundheitsdienstleister die gesamte Datenbank zur Verfügung und kann die ihn zweckmäßig erscheinenden Medizindaten auswählen, die dann in den Barcode kodiert und so dem Gesundheitsdienstleister zur Übertragung bereitgestellt werden.

Sicherheitsbedenken hinsichtlich eines unautorisierten Zugriffs auf den Datenspeicher oder die Medizindaten bestehen damit nicht. Zum Eingeben von Medizindaten umfasst das Mobiltelefon oder Tablet eine Barcode-Leseeinrichtung, z.B. über die Kamerafunktion des Gerätes. Das Computerprogramm hat ein Eingabemodul, das einen Barcode mittels der Barcode-Leseeinrichtung einliest. Darin enthaltene Medizindaten werden aus dem Barcode dekodiert und in der Datenbank abgelegt. Finden sich in einem Barcode keine gültigen Medizindaten, kann eine Fehlermeldung ausgegeben werden, oder die eingelesenen Daten werden unmittelbar von der App angezeigt, so dass der Patient prüfen kann, ob der eingelesene Text korrekt ist, z.B. mit einem ausgedruckten Arztbrief übereinstimmt. Damit ist auch für den Fall Vorsorge getroffen, dass ein Benutzer irrtümlich einen Barcode einliest, der gar keine Medizindaten enthält.

Eine Vorrichtung zum Verwalten von auf den Patienten bezogenen Medizindaten kann dann als Mobiltelefon oder Tablet ausgebildet sein, das einen Bildschirm, eine Eingabevorrichtung, einen Datenspeicher und ein Computerprogramm der geschilderten Art aufweist.

Ein andersartiger Zugang zum Einpflegen von Medizindaten in die Datenbank ist natürlich gleichermaßen möglich. So ist es möglich, dass ein Patient selbst die Daten manuell eingibt, beispielsweise wenn der Gesundheitsdienstleister keinen Barcode bereitstellte. Hier ist es zu bevorzugen, Zugriff auf die Datenbank im Mobiltelefon oder Tablet von einem Computer aus zu nehmen, über den ebenfalls der Patient die Verfügungsmacht hat. Solche geschützten Zugriffe auf Mobiltelefone oder Tablets sind im Stand der Technik vielfältig bekannt und werden von Herstellern von Mobiltelefonen oder Tablets beziehungsweise den Betriebssystemproduzenten bereitgestellt.

Natürlich können Medizindaten auch auf Seiten des Patienten erfasst werden, z. B. regelmäßige Temperatur- oder Blutdruckmessungen. Entsprechende Messgeräte können einen Barcode bereitstellen, der dann vom Patienten zum Einlesen in die Datenbank verwendet wird. Alternativ ist auch eine direkte Datenverbindung mit dem Mobiltelefon möglich, beispielsweise über den Standard Bluetooth. Auf diese Weise kann der Patient seine Datenbank um selbst erfasste Messdaten anreichern.

Der Barcode ist ein beliebiger, optoelektronisch erfassbarer Schriftcode. Beispiele hierfür sind 1D-Barcodes, Strichcodes oder 2D-Barcodes, insbesondere Datamatrix-Codes oder QR-Codes. Aus Sicherheitsgründen ist es bevorzugt, bei der Kodierung und/oder Dekodierung des Barcodes nur druckbare Zeichen oder Zeilenschaltungen zu berücksichtigen. Auf diese Weise ist gewährleistet, dass unerwünschte Steuerbefehle, welche am einlesenden Gerät eine nicht gewünschte oder überblickbare Wirkung haben könnten, gar nicht aus dem Barcode dekodiert oder in diesem kodiert werden. Diese Filterung ist ein weiteres Sicherheitskriterium.

In Ausgestaltungen ist es vorgesehen, dass ein Benutzer Medizindaten in die Datenbank eingibt. Die App verfügt hierzu über ein entsprechendes Eingabemodul und die Vorrichtung über eine entsprechende Eingabevorrichtung, beispielsweise eine Bildschirmtastatur. Dies ist besonders vorteilhaft, wenn der Einsatz in Form eines elektronischen Klemmbretts erfolgt. Der Benutzer, beispielsweise der Patient, gibt zur Anamneseerhebung in die Datenbank, die auf einem Tablet vorgesehen ist, welches ihm zu Beginn des Arztbesuches ausgehändigt wurde, Medizindaten ein. Hierbei kann beispielsweise ein entsprechender Fragebogen vorgesehen werden, den der Patient abarbeitet. Anschließend übergibt er das Tablet an eine Praxiskraft, welche die richtigen und/oder relevanten und/oder anderen Kriterien genügende Medizindaten auswählt, so dass diese in einen Barcode kodiert und dadurch in die elektronische Patientenakte des Arztes übertragen werden können. Gleichermaßen kann das elektronische Klemmbrett von einer Praxiskraft auch während einer Untersuchung verwendet werden, um Untersuchungsergebnisse, beispielsweise Körpergröße, Körpergewicht etc. einzutragen, dann diese Medizindaten aus der derart befüllten Datenbank auswählen und per Barcode in die elektronische Patientenakte des Arztes übertragen kann.

Zusätzlich ist es in Weiterbildungen möglich, für Medizindaten eine digitale Signatur zu erzeugen. Diese stellt ein weiteres Medizindatum dar und bezieht sich auf Richtigkeit und Quelle von Medizindaten in der Datenbank. Diese Signatur wird bevorzugt mit den davon betroffenen Medizindaten in den Barcode kodiert, so dass der Empfänger Information über Richtigkeit und Quelle der Medizindaten erhält. Bei der Verwendung als elektronisches Klemmbrett durch eine Praxiskraft kann die Signatur automatisch generiert und dann in den Barcode inkludiert werden.

Elektronische Patientenakten arbeiten weitverbreitet mit Freitextfeldern. Dies hat den Vorteil, dass beliebige Information eingegeben werden kann. Nachteilig ist eine fehlende Übersichtlichkeit und die schwere EDV-Auslesbarkeit. Auch ist dann keine strukturierte Weiterverarbeitbarkeit in Zielsystemen möglich. Es ist deshalb in Weiterbildungen vorgesehen, in den Barcode bezüglich der Medizindaten verschiedene Strukturierungszeichen einzufügen, welche beispielsweise Feldtitel, Felddaten und Datengruppen voneinander unterscheidet. Dann ist nicht nur eine Strukturierung möglich, sondern Medizindaten können gemäß der Strukturierung auch in verschiedene Barcodes kodiert und so getrennt und/gruppiert ausgegeben werden. Das erschließt eine Anpassung an verschiedene Arten von Zielsystemen, z.B. elektronische Patientenakten, Qualitätssicherungsdokumentationen, wissenschaftliche Studiendatenbanken.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: ein Verfahrensablaufdiagramm zum Übertragen von Medizindaten von einem Patienten zu einem Arzt,
- Fig. 2: ein Ablaufdiagramm für ein Verfahren zum Übertragen von Medizindaten vom Arzt zum Patienten,
- Fig. 3: eine Schemadarstellung der wesentlichen Funktionsblöcke einer Vorrichtung zur Barcodeübertragung und zur Verwaltung von Medizindaten,
- Fig. 4: eine Schemadarstellung einer Softwarestruktur der Vorrichtung der Fig. 3,
- Fig. 5: ein Beispiel für einen erzeugten Barcode,
- Fig. 6: ein System bestehend aus der Vorrichtung der Fig. 3 sowie Datenaustausch- und Speicher-Elementen auf Seiten des Arztes,
- Fig. 7: ein Überblick über den Einsatz der App mit der Vorrichtung der Fig. 3,
- Fig. 8: ein Überblick über eine mögliche Datenerfassung verschiedener Arten von Medizindaten mit der App,
- Fig. 9: ein Beispiel für die Bereitstellung von Medizindaten als Barcode und
- Fig. 10: ein Beispiel für das Einscannen von Patientendaten als Medizindaten.

Der Patient erfasst verschiedenste Arten von ihn betreffenden Medizindaten mit einer App auf seinem Mobiltelefon. Beim Arztbesuch überträgt er von ihm zuvor ausgewählte Daten mittels der App. Dazu kann er Filter einsetzen (z.B. nur Labordaten). Für die ausgewählten Daten erzeugt die App einen Barcode, der über einen Barcode-Scanner in das IT-System des Arztes eingelesen wird. Soweit nachfolgend auf Ärzte Bezug genommen wird, ist das nur stellvertretend für Gesundheitsdienstleister zu verstehen.

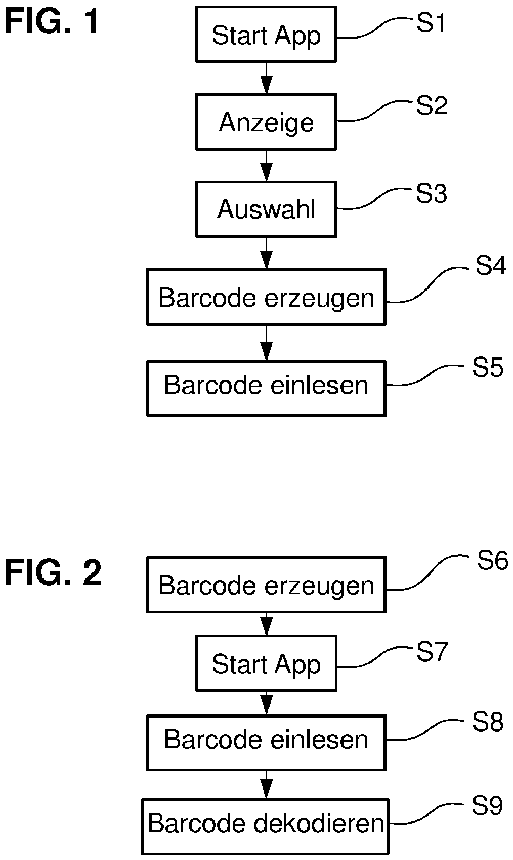

Fig. 1 zeigt ein schematisches Ablaufdiagramm dieses Verfahrens. In einem Schritt S1 ruft der Patient eine App auf seinem Mobiltelefon auf. In einem Schritt S2 zeigt ihm die App daraufhin eine Übersicht über Medizindaten, welche in einer Datenbank gespeichert sind, die die App auf dem Mobiltelefon verwaltet. Bei den Medizindaten kann es sich um Messwerte, in einem Labor erfasste Werte, Befunde oder Diagnosen handeln, die sich sämtlich auf eine Person beziehen, nämlich den Besitzer des Mobiltelefons. Er hat diese Daten auf noch zu erläuternde Art und Weise gesammelt und in der Datenbank zusammengetragen. Im Schritt S2 wird ihm eine Übersicht angezeigt, die ihm eine Grundinformation für jeden Datensatz gibt, der abgelegt ist. Der Patient kann so entscheiden, welche Medizindaten dem Arzt zur Verfügung gestellt werden sollen. Er kann diese Entscheidung mit seinem Arzt oder dessen Mitarbeiter besprechen. Sodann wählt der Patient in einem Schritt S3 die zu übertragenden Medizindaten aus. In einem Schritt S4 kodiert die App die ausgewählten Medizindaten in einen Datamatrix-Code und zeigt diesen auf dem Bildschirm des Mobiltelefons an. In einem Schritt S5 gestattet der Patient dem Arzt oder dessen Mitarbeiter das Einlesen des Barcodes. Der Barcode enthält reine Textinformation, also ausschließlich druckbare Zeichen und Zeilenschaltungen. Der Arzt benötigt deshalb kein besonderes System, da das Einlesen des Barcodes für ihn keine andere Dateneingabe darstellt, als es die Eingabe über eine Tastatur wäre. Sofern die Arztsoftware leistungsfähig ist, werden auch strukturierte Daten (z.B. im XML-Format oder JSON-Format) via Barcode übertragen. Der Patient ist Herr der Entscheidung, ob Medizindaten für die Übertragung bereitgestellt werden, ob also überhaupt eine Übertragung stattfindet, und in welchem Umfang Medizindaten übertragen werden sollen.

Umgekehrt kann derselbe Arzt oder ein anderer Arzt, ein Labor etc. Daten für den Patienten als Barcode bereitstellen, die der Patient per App einlesen kann. Hierzu wird in einem Schritt S6 vom Arzt ein Barcode erzeugt, der die bereitzustellenden Medizindaten kodiert. Dieser Barcode kann beispielsweise auf einem Arztbrief oder Laborbericht gedruckt sein. Der Patient startet daraufhin in einem Schritt S7 die App und liest über die am Mobiltelefon vorhandene Kamera den Barcode in einem Schritt S8 ein. Die App dekodiert den Barcode daraufhin in einem Schritt S9 und extrahiert dabei die darin niedergelegten Medizindaten. Findet er keine zulässigen Medizindaten, gibt die App eine Fehlermeldung aus. Dies kann beispielsweise der Fall sein, wenn der Patient irrtümlich einen falschen Barcode scannte. Dies ist jedoch die Ausnahme und die Dekodierung führt im Schritt S9 im Regelfall dazu, dass Medizindaten erhalten werden und von der App in die Datenbank eingestellt werden. Die eingelesenen Daten werden optional danach angezeigt, so dass der Patient ihre Richtigkeit prüfen kann, ob z.B. der ausgedruckte Bericht mit dem von der App in die Datenbank eingelesenen Text übereinstimmt.

Um eine unerwünschte Beeinflussung des IT-Gerätes, welches den Barcode einliest, zu verhindern, ist sowohl bei Kodierung als auch bei Dekodierung vorgesehen, dass nur druckbare Zeichen und Zeilenschaltungen extrahiert werden. Andere Zeichen, insbesondere Tabulatorzeichen, die als Steuerbefehle wirken könnten, werden ausgefiltert und bei der Kodierung oder Dekodierung nicht mit übernommen. Somit ist auf beiden Seiten, d.h. sowohl auf Seiten des ärztlichen IT-Systems als auch auf Seiten des Mobiltelefons eine unerwünschte Beeinflussung durch den eingelesenen Barcode ausgeschlossen.

Die Verwendung des Barcodes hat den Vorteil, dass es keinen Rückkanal gibt. Der Barcode kann nur diejenigen Medizindaten übertragen, die bei seiner Erzeugung verwendet wurden. Da die Ausgabe aus der Datenbank, die der Patient über seine App im Mobiltelefon pflegt, zum Arzt hin durch einen Barcode erfolgt, welcher von der App nur auf Anforderung durch den Patienten und nur für Medizindaten erzeugt wird, die der Patient freigeben möchte, ist ein unerwünschtes Auslesen oder gar Ausspähen von Medizindaten in der Übertragung vom Patient zum Arzt ausgeschlossen. Das gleiche gilt auch in der anderen Richtung. Der vom Arzt bereitgestellte Barcode enthält nur Medizindaten, welche beispielsweise dem Text des Arztbriefes entsprechen, die der Arzt weitergeben möchte. Ein unerwünschter Zugriff auf Medizindaten anderer Patienten ist damit ausgeschlossen.

In einer besonders zweckmäßigen Ausführungsform kodiert der Barcode, z.B. Datamatrix-Code, welcher auf dem Arztbrief oder dem Laborbefund etc. aufgedruckt ist, den dort gedruckten Text. Eine derartige Funktion ist in üblichen Textverarbeitungsprogrammen vorhanden und kann somit ohne weitere Anforderungen an Software oder Hardware des ärztlichen IT-Systems realisiert werden.

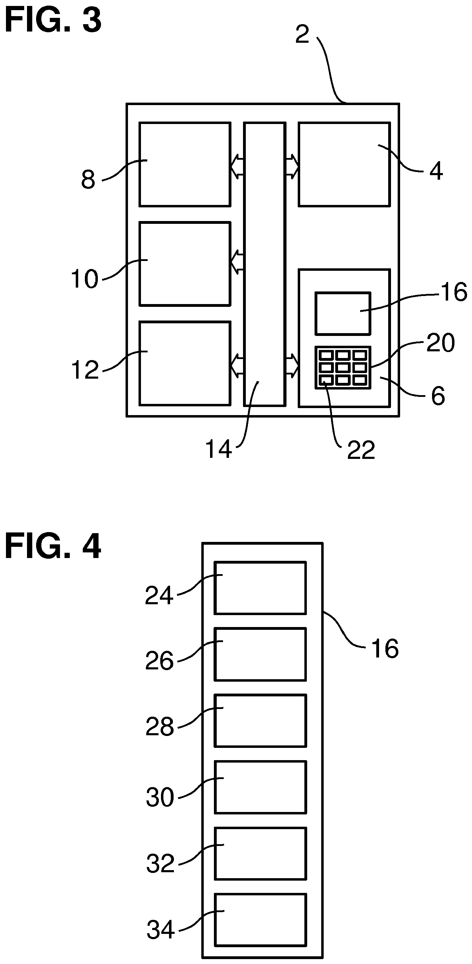

Fig. 3 zeigt schematisch eine Vorrichtung zum Verwalten von Medizindaten. Sie ist in diesem Fall als Mobiltelefon 2 ausgebildet, kann jedoch auch in Form beliebiger anderer portabler Geräte, beispielsweise in Form eines Tablet ausgebildet werden. Das Mobiltelefon 2 umfasst neben anderen, für die vorliegenden Funktionen nicht relevanten Elementen einen Prozessor 4, der auf einen Speicher 6 zugreift. Weiter umfasst das Mobiltelefon 2 einen Bildschirm 8, eine Eingabevorrichtung 10 in Form einer Touch-Screen-Funktionalität des Bildschirms 8 sowie eine Kamera 12. Diese Komponenten sind mit dem Prozessor 4 über einen Datenbus 14 verbunden. Neben einem nicht weiter relevanten Betriebssystem, das beispielsweise das bekannte Betriebssystem Android oder iOS sein kann, befindet sich im Speicher Programmcode (z.B. in Java oder C++ geschrieben) für eine App 16. Diese verwaltet im Datenspeicher 6 des Mobiltelefons eine Datenbank 20, in welcher eine Vielzahl an Medizindaten 22 abgespeichert ist.

Die Ausgabe von Medizindaten an einen Arzt oder sonstigen medizinischen Dienstleister erfolgt mit dem Verfahren gemäß Fig. 1. Unter Steuerung der App 16 zeigt der Prozessor 4 am Bildschirm 8 eine Übersicht über die Medizindaten 22 an. Der Patient wählt einzelne Medizindaten 22 aus, woraufhin der Prozessor 4 unter Steuerung der App 16 diese Medizindaten in einen QR-Code kodiert und diesen auf der Anzeige 8 darstellt. So kann der Arzt oder medizinische Dienstleister mit einem einfachen Barcodeleser die vom Patienten zur Verfügung gestellten Medizindaten 22 in sein IT-System übertragen.

Auch die Eingabe von Medizindaten 22 in die Datenbank 20 erfolgt mind. teilweise unter Steuerung der App 16 basierend auf Barcodes. Der Arzt stellt einen Barcode bereit, und das Mobiltelefon 2 wird vom Patienten dazu genutzt, unter Steuerung der App 16 mittels der Kamera 12 den Barcode einzulesen. Der Prozessor 4 dekodiert den Barcode und extrahiert die Medizindaten 22 und stellt sie in die Datenbank 20 ein.

Fig. 4 zeigt schematisch die App 16, deren Programmcode bei Ausführung auf dem Prozessor 4 verschiedene Software-Module bereitstellt. Ein Datenbankmodul 24 dient zur Verwaltung der Medizindaten 22 in der Datenbank 20. Ein Auswahlmodul 26 ist so ausgebildet, dass es dem Benutzer die Auswahl der in den Barcode zu übernehmenden Medizindaten 22 ermöglicht. Es steuert dazu den Bildschirm 8 geeignet an, so dass eine Übersicht über die in der Datenbank 20 vorhandenen Medizindaten 22 gegeben wird und über die Eingabeeinrichtung 10 eine Auswahl der zur übertragenden Medizindaten 22 erfolgen kann. Ein Kodierungsmodul 28 kodiert Medizindaten 22 in einen Barcode oder dekodiert einen Barcode und extrahiert daraus Medizindaten 22. Wie bereits erwähnt, werden dabei Steuerzeichen unterdrückt, so dass nur druckbare Zeichen und Zeilenschaltungen in den Barcode kodiert bzw. aus dem Barcode extrahiert werden. Ein Übertragungsmodul 30 ist ausgebildet, einen vom Kodiermodul 28 erzeugten Barcode auf der Anzeigevorrichtung 8 anzuzeigen. Ein Eingabemodul 32 ist ausgebildet, einen Barcode 36 mittels der Kamera 12 des Mobiltelefons 2 einzulesen und an das Kodiermodul 28 zu Dekodierung zu übergeben.

Die App 16 verfügt weiter noch über ein Verbindungsmodul 34, mit dem das Mobilfunkgerät 2 über eine nicht weiter bezeichnete Schnittstelle, beispielsweise eine Bluetoothschnittstelle eine Verbindung zu einem Messgerät, beispielsweise einem Fieberthermometer, einem Blutdruckmessgerät oder einem Blutzuckermessgerät aufnehmen kann, um von diesem Messgerät Medizindaten 22 zu übernehmen und in die Datenbank 20 einzustellen. Diese Funktionalität der App 16 ermöglicht es dem Patienten, eigenständig Medizindaten 22 aufzunehmen, beispielweise im Rahmen einer kontinuierlichen Überwachung, wie sie bei bestimmten Erkrankungen indiziert sein kann.

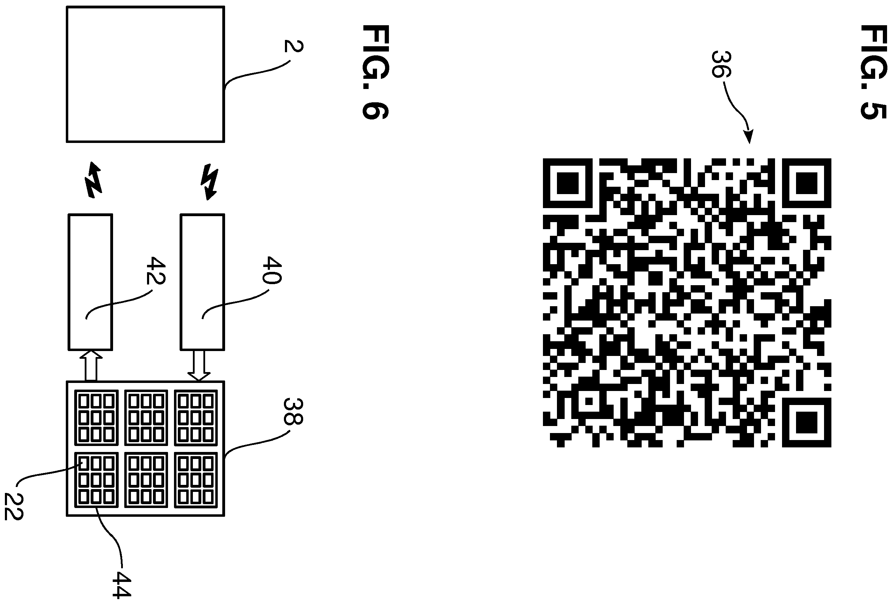

Fig. 5 zeigt exemplarisch einen QR-Code 36, wie er zur Übertragung der Medizindaten 22 verwendet werden kann.

Fig. 6 zeigt schematisch ein System 36, das ein Mobiltelefon der beschriebenen Art sowie eine Datenbank 38 eines Gesundheitsdienstleisters umfasst, in der für eine Vielzahl von Patienten Medizindaten 22 gespeichert sind. An die Datenbank 38 ist eine Barcode-Lese- und Dekodiervorrichtung 40 angeschlossen. Diese liest einen vom Mobiltelefon 2 bereitgestellten Barcode ein. Der Pfeil zwischen der Vorrichtung 40 und dem Mobiltelefon 2 symbolisiert zum einen, dass dieser Datenübertragungsvorgang nur in eine Richtung erfolgen kann, nämlich vom Mobiltelefon 2 zur Vorrichtung 40 hin. Er symbolisiert weiter, dass die Übertragung kontaktlos erfolgt, also ohne, dass es einer Netzwerkverbindung etc. bedürfte. Die Barcode-Lese- und Dekodiervorrichtung 40 liest den Barcode ein und dekodiert ihn. Auf diese Weise extrahiert sie die Medizindaten 22 aus dem Barcode 36 und speichert ihn in einem Datensatz 44, der dem entsprechenden Patienten zugeordnet ist. Da der Barcode 36 mehrere Medizindaten 22 enthalten kann, können auf diesem Weg in einem Schritt mehrere Medizindaten an den behandelnden Arzt, d.h. dessen Datenbank 38 übertragen werden.

Vom Gesundheitsdienstleister bereitgestellte bzw. erstellte Medizindaten, beispielsweise Befunde, Laborberichte, Arztbriefe, Diagnosen etc., werden aus der Datenbank 38 von einer Medizindaten-Kodier- und -ausgabevorrichtung 42 ausgelesen. Dies kann beispielsweise dadurch erfolgen, dass der Text eines üblichen Arztbriefes in einen Barcode 36 kodiert wird. Er wird dem Patienten ausgegeben, so dass dieser ihn mit seinem Mobiltelefon 2 einlesen kann. Beispielweise wird der Barcode auf den Arztbrief aufgedruckt, dem Patienten in einem separaten Druckstück mitgegeben oder auf einem Bildschirm angezeigt, wo der Patient den Barcode mit seinem Mobiltelefon 2 auf die beschriebene Art und Weise einlesen kann.

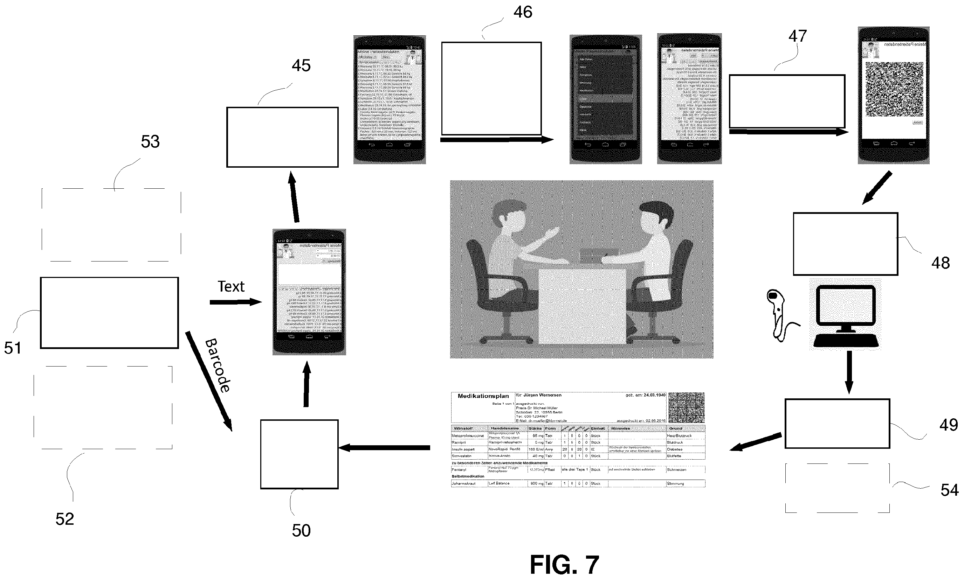

Fig. 7 zeigt schematisch einen möglichen Ablauf, wie die App 16 zur Sammlung von Patientendaten, zum Übertragen an einen Arzt und zur Aufnahme von weiteren Patientendaten genutzt werden kann.

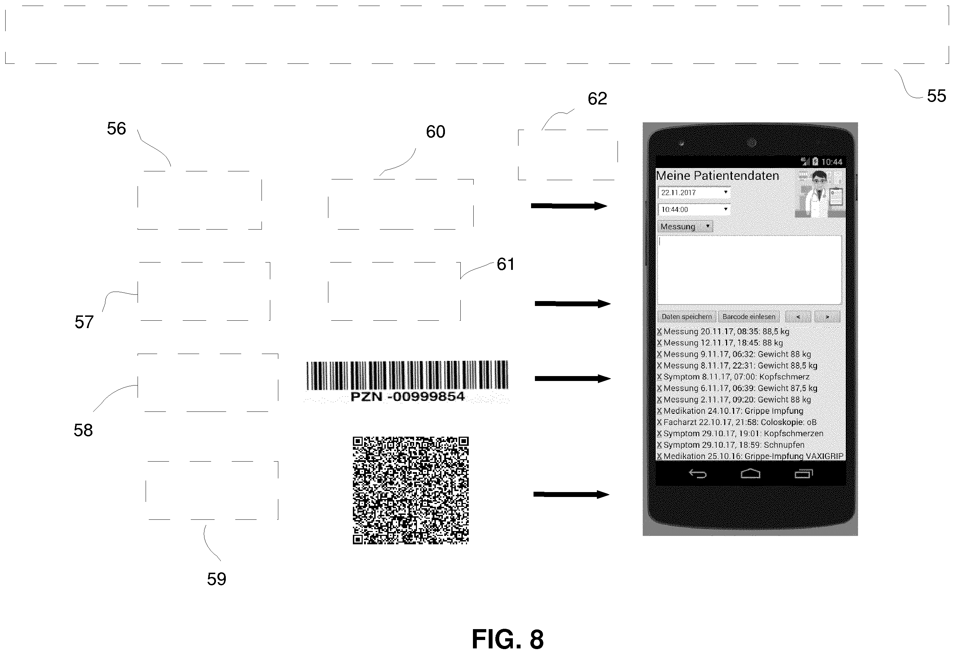

Fig. 8 zeigt, welche Arten von Datenerfassung mit Ausführungsformen der App 16 vorgesehen sind. Der Patient kann die Eingabe sowohl über die Tastatur von Smartphone bzw. Tablet vornehmen als auch über Barcodes (sowohl 2D-Barcodes, insbesondere QR-Codes oder Datamatrix-Codes, als auch 1D-Barcodes, beispielsweise PZN-Nummern von Arzneimitteln) oder eine Verbindung zu Messgeräten über das Verbindungsmodul 34. Die Einträge werden automatisiert mit Zeitstempel versehen (z.B. um den Zeitpunkt von Symptomen oder Messungen zu dokumentieren), sodass später der zeitliche Verlauf beispielsweise von Symptomen oder Messungen für den Arzt ersichtlich ist. Den Einträgen werden optional Kategorien (z.B. Messung, Symptom, Labor) zugeordnet, um das spätere Filtern der Daten zu unterstützen. Die App 16 enthält optional weiter eine Datenbank von Arzneimitteln, sodass aus einer PZN-Nummer (z.B. 00999854) automatisch der Wirkstoff (z.B. Xylometazolin), Handelsname/Darreichungsform (z.B. Nasenspray Ratiopharm KDR) ermittelt werden. Verschiedene Kategorien von Daten (z.B. Symptome, Messungen, Medikation) können sowohl als Text wie auch als Barcode eingelesen werden.

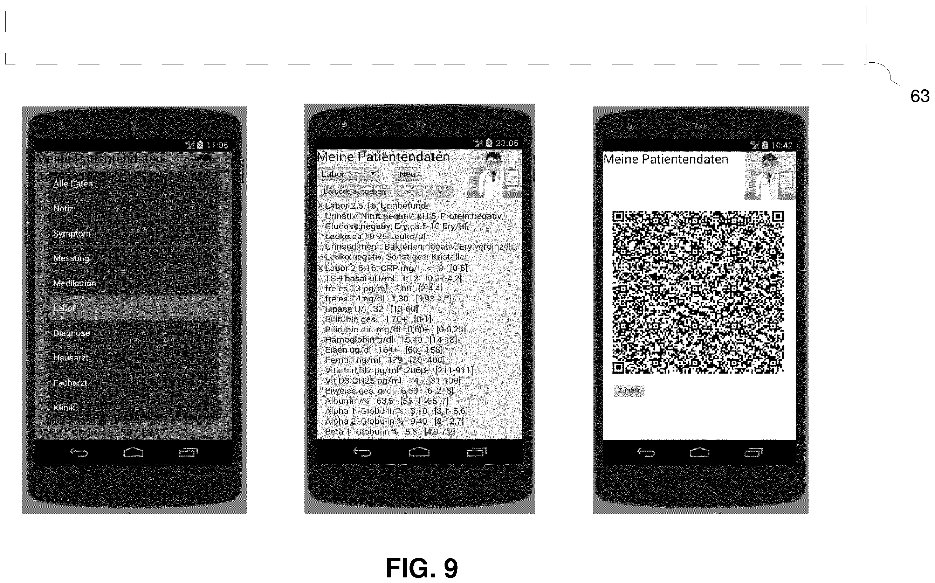

Fig. 9 zeigt, wie die Daten in der App zur Ausgabe gefiltert werden können. Die Auswahl einer Kategorie (hier "Labor") liefert eine Vorauswahl der Daten (linkes Bild). Der Patient wählt ggf. weiter aus, z.B. einen Zeitraum (mittleres Bild). Aus diesen Daten erstellt die App 16 den Barcode 36, der mit einem handelsüblichen Barcode-Scanner in ein nahezu beliebiges IT-System des Arztes eingelesen werden kann (rechtes Bild). Barcode-Scanner sind bevorzugt so konfiguriert, dass sie sich im ärztlichen IT-System wie eine Tastatur verhalten. Eine Eingabe via Tastatur ist in nahezu beliebige IT-Systeme möglich.

Der Datenaustausch erfolgt bei jeder Ausführung rein unidirektional, im Ergebnis des Ablaufs aber durchaus bidirektional: Der Patient liefert per Barcode 36 Daten 22, er erhält aber per Barcode 36 auch Daten vom Arzt, zum Beispiel Untersuchungsdaten, Befunde, Diagnosen, Laborbefunde oder einen Medikationsplan. Dies erfolgt mitunter bei ein und demselben Arztbesuch.

Der in Deutschland bundeseinheitliche Medikationsplan sieht einen standardisierten Barcode vor, sodass die Angaben des Medikationsplans direkt mittels der App 16 eingelesen werden können. Dieser Medikationsplan liegt im sogenannten Ultrakurzformat vor, da nur PZN-Nummern der Medikamente enthalten sind. Durch eine optionale lokale Datenbank in der App 16 werden diesen PZN-Nummern Wirkstoff-/Handelsnamen und Darreichungsformen zugeordnet, sodass sie aus einem nur mit Computer verarbeitbaren Format in für den Patienten verwendbare Medizindaten 22 umgewandelt und in seiner Datenbank 20 abgelegt werden. Bei IT-Systemen, die Barcode-Erzeugung nicht direkt unterstützen, können die Texte beispielsweise über die Zwischenablage (Clipboard) eines Computers in den Barcode-Generator 40 kopiert werden und damit der Barcode 36 erzeugt werden, den der Patient in die App 16 einlesen kann. Allgemeine Informationstexte, z.B. Diäthinweise oder krankheitsbezogene Patienteninformationen, können als Infoblätter mit Barcode 36 vom Arzt bereitgestellt werden, sodass der Patient via Barcode 36 diese Informationen direkt in seine App 16 laden kann. Auch dies sind Medizindaten im hier verstandenen Sinn.

Verschiedene Barcode-Typen, insbesondere QR-Codes, Datamatrix-Codes und 1D-Barcodes können von der App 16 eingelesen und/oder erzeugt werden.

Je nach Umfang der zu übertragenden Medizindaten kann es zweckmäßig sein, die Kodierung auf mehrere Barcode-Elemente aufzuteilen. Bei der Übertragung von Medizindaten in das IT-System eines Arztes kann beispielsweise es erforderlich werden, eine große Menge an Medizindaten von der App 16 bereitzustellen. Der Patient sollte im Umfang, in dem er Medizindaten bereitstellen kann, möglichst nicht eingeschränkt sein. Es ist deshalb bei einer Weiterbildung eine maximale Zeichendichte für den Barcode 36 vorgegeben, so dass die App 16 automatisch jedes Barcode-Element nur bis zur gewünschten Zeichendichte mit Information belegt. Diese Ausgestaltung trägt der Tatsache Rechnung, dass ab gewissen Zeichendichten Barcodes, insbesondere 2D-Barcodes, mitunter schwer lesbar werden oder es zu Fehleinlesungen kommen kann. Dann stellt in App 16 zuerst die zu übertragenden Medizindaten 22 zusammen und kodiert diese dann je nach Datenmenge in einen oder mehrere Barcode-Elemente 36, wobei erst das Einlesen aller Barcode-Elemente die vollständige Menge der ausgewählten Medizindaten 22 liefert. Die Erzeugung des Barcodes 36 auf Anforderung durch den Patienten stellt dabei sicher, dass exakt die gewünschten Medizindaten 22 in Barcode-Elemente umgesetzt werden. Eine mögliche Obergrenze für die Informationsdichte im Barcode 36 liegt zwischen 2000 und 4000 kodierten Zeichen pro Barcode. Oberhalb dieses Bereichs, insbesondere bevorzugt oberhalb von 4000 Zeichen, besonders bevorzugt 3000 Zeichen und ganz besonders bevorzugt 2000 Zeichen, ist ein zuverlässiges Einlesen des Barcodes stark von der Qualität des Mediums, auf dem der Barcode bereitgestellt wird, und des Barcode-Lesers abhängig. Das Einhalten dieser Obergrenze und das Aufteilen in eine Serie von Barcode-Elementen vermeidet Probleme, die sich aus zu hoch komprimierten Barcodes ergeben könnten.

Die App 16 hat insbesondere folgende Eigenschaften/Merkmale:
1) Patientendaten sind unter der Kontrolle des Patienten. Die Medizindaten 22 werden optional auf dem Smartphone/Tablet 2 in verschlüsselter Form gespeichert. Der Patient entscheidet, wem er welchen Teil seiner Medizindaten 22 zeigt (z.B. über eine Filterfunktion nach Kategorien bzw. über die Wahl eines Zeitausschnitts). Dadurch werden die Vorgaben des Datenschutzes eingehalten.
2) Die Medizindaten 22 befinden sich immer beim Patienten, und zwar vollständig, auch wenn mehrere Ärzte in die Behandlung einbezogen sind. Dies erhöht die Patientensicherheit (vor Behandlungsfehlern durch fehlende Informationen oder inkompatiblen Arzneimitteltherapien), vermeidet Doppeluntersuchungen und optimiert die Therapie.
3) Der Datenaustausch Patient->Arzt sowie Arzt->Patient erfolgt über Barcodes 36. Dadurch werden die Medizindaten sehr zeitsparend (wenige Sekunden) und korrekt (keine Tipp-Fehler) in ein nahezu beliebiges IT-System des Arztes (sowohl Arztpraxissysteme als auch klinische Arbeitsplatzsysteme) übermittelt bzw. in die App 16 des Patienten übertragen. Die Übermittlung in IT-Systeme des Arztes stellt einen besonders wichtigen Vorteil da, weil es sehr viele, untereinander nicht kompatible IT-Systeme für Ärzte gibt und diese IT-Systeme aus Gründen von Datenschutz und Datensicherheit stark technisch abgeschirmt sind.
4) Der Datenaustausch erfolgt als Standard nicht über Daten-Netzwerke, insbesondere nicht über das Internet bzw. Cloud-Services, sondern rein optisch per Barcode. Dadurch wird ein sehr hohes Schutzniveau erreicht (kein Abhören/Kopieren von Daten beim Transfer möglich). Dies schließt nicht aus, dass zusätzlich größere Datenmengen, beispielsweise Bilderserien, über herkömmliche Datenverbindungen (z.B. Bluetooth oder IP-basierte Netze) übertragen werden. Um hierzu die Kontrolle durch den Patienten sicherzustellen, ist es in einer Erweiterung der App 16 vorgesehen, über den Barcode Einmal-URLs zu übertragen, mit denen der Zugriff auf größere Datenmengen ermöglicht wird, die auch für eine Serie von Barcodes zu groß wären. Mit dieser Erweiterungsvariante würde zusätzlich zur Übertragung von Medizindaten per Barcode auch eine netzbasierte Übertragung von Zusatzdaten durchgeführt.
5) Die Authentifizierung/Autorisierung und Bedienung der App 16 ist einfach und sicher: Der Zugang zu den Medizindaten funktioniert genauso wie der Zugang zum Smartphone 2 bzw. Tablet. Diese Geräte werden von den Patienten sehr häufig für vielfältige Aufgaben genutzt und sind ihnen daher sehr vertraut. Den Zugang zu seinem Smartphone/Tablet kann der Patient mit etablierten Techniken schützen (z.B. PIN, Muster, Gesichtserkennung, Remote-Löschung bei Diebstahl des Smartphones etc.). Andere Authentifizierungsverfahren für Medizindaten, z.B. die Eingabe von speziellen PIN beim Arztbesuch, haben sich hingegen in der Praxis als untauglich erwiesen, weil die Patienten diese PIN häufig vergessen. Es ist ein Spezifikum für elektronische Systeme in der Medizin, dass diese für die Patienten sehr einfach und zuverlässig bedienbar sein müssen, weil ein Großteil der Patienten durch die jeweilige Erkrankung oder altersbedingt in seinen Handlungsmöglichkeiten eingeschränkt ist.
6) Die Medizindaten werden mit sehr hoher Vollständigkeit und Präzision beim zeitlich kurzen Arztbesuch in elektronischer Form übermittelt. Beispielsweise die Fragen: Wie waren Ihre Schmerzen in den letzten 2 Wochen? Oder wie waren Ihre Blutdruckwerte im letzten Monat? Können mit Hilfe der App quantitativ und präzise beantwortet werden, und dies in sehr kurzer Zeit. Durch eine optional vorgesehene automatische Vergabe von Zeitstempeln für jeden Eingabevorgang von Medizindaten 22 in der App 16 werden Eingaben (z.B. Symptome) korrekt zeitlich zugeordnet. Der Patient kann sich auf den Arztbesuch durch Eingabe seiner Medizindaten in die App 16 sehr gut vorbereiten und damit sicherstellen, dass trotz kurzer Zeit keine wichtigen Angaben vergessen werden.
7) Durch die optionale Filterfunktion können bestimmte Datenkategorien, zum Beispiel Laborwerte oder Diagnosen, sehr schnell aufgefunden werden. Dies ist insbesondere bei Patienten mit chronischen Erkrankungen, die sehr viele Medizindaten aufweisen, von Bedeutung. Fehler durch Übersehen von Vorerkrankungen oder Allergien werden vermieden; der Verlauf von Laborparametern kann besser geprüft werden; Doppeluntersuchungen können vermieden werden.
8) Der Arzt kann dem Patienten relevante Informationen, z.B. Arztbrief, Laborwerte, Medikation, Patienteninformationen, in elektronischer Form als Medizindaten 22 schnell und effizient bereitstellen.
9) Der in Deutschland bundeseinheitliche Medikationsplan wird optional von der App 16 unterstützt. Dessen Barcodes können von der App 16 eingelesen und wieder exportiert werden.
10) Die Arzneimitteltherapie des Patienten wird unterstützt: Durch das optionale Einscannen und Dekodieren von PZN-Codes, die auf Verpackungen von Arzneimitteln angebracht sind, kann der Patient einfach und schnell die Einnahme von Arzneimitteln und Zusatzinformationen (z.B. zu Wirkungen oder Nebenwirkungen) dokumentieren.
11) Der Patient kann seine Medizindaten sichern, indem er via Kabel die optional verschlüsselte Datenbank 20 auf einem anderen Computer sichert. Die Medizindaten 22 sind hierbei optional so verschlüsselt, dass sie nur von der App 16 entschlüsselt werden können. Auf diese Weise kann der Patient seine Medizindaten auf ein neues Endgerät übertragen.
12) Der Datenbestand der App 16 ermöglicht eine Reihe von Erweiterungen, die durch die App 16 wirksam unterstützt werden: App-basierte Erinnerungsfunktionen zur Einnahme von Medikamenten, basierend auf dem jeweiligen Medikationsplan (zur Erhöhung von Adhärenz/Compliance in der Arzneimitteltherapie); Bereitstellung von Notfallinformationen auf dem Smartphone/Tablet 2 (z.B. auf der Startseite); Erfassung von Patientenfragebögen (z.B. Patient Reported Outcomes), die in die elektronische Patientenakte integrierbar sind; Erfassung von Fotos und anderen Medien in der App 16, zusätzlich zu Kategorie und Zusammenfassung, beispielsweise Fotos von Hautläsionen, Verletzungen, Röntgenbilder, endoskopische oder mikroskopische Bilder; Bereitstellung von Medizindaten 22 in anonymisierter Form für wissenschaftliche Zwecke, sofern der Patient zustimmt; multilinguale Version der App 16, damit der Patient in seiner Muttersprache seine Medizindaten 22 erfassen und verwalten kann.

Aus den beschriebenen Ausführungsformen wird deutlich, dass das Verfahren und die offenbarte Vorrichtung auf verschiedene Weisen verwirklicht werden können. Die oben beschriebenen Ausführungsformen dienen lediglich der Veranschaulichung. Beispielsweise kann eine andere Aufteilung der Software-Module verwendet werden. Darüber hinaus kann die Hardwareausstattung des Mobiltelefons/Tablets vom beschriebenen Umfang abweichen, solange die notwendigen Funktionen erfüllt werden. Insbesondere kann das Tablet ohne Mobilfunkfähigkeit ausgeführt werden.

Für den Fachmann ist es klar, dass einige oder alle Schritte der beschriebenen Verfahren durch Hardware realisiert werden kann/können, welche der Anweisung eines Programms folgt/folgen. Die App kann in Form von Programmcode in einem computerlesbaren Speicher abgelegt sein und die Schritte der oben beschriebenen Verfahren ausführen, wenn der Programmcode auf einem geeigneten Gerät (z.B. dem genannten Mobiltelefon/Tablet) installiert und dann ausgeführt wird. Der Speicher kann ein beliebiges physisches Speichermedium zum Speichern von Programmcode (z.B. ein mobiler Datenträger, eine Speicherkarte) oder ein über ein Netzwerk zugänglicher Speicher sein. Die App kann insbesondere über Online-Stores (z.B. Google Play Store) abrufbar bereitgestellt werden. Auch können App und/oder Datenbank auf einem oder mehreren, logisch eingebundenen externen Speichern, z.B. Cloud-Speicher, abgelegt sein. Programm und Datenbank können auf demselben oder verschiedenen Speichern abgelegt sein. So kann z.B. die App 16 auf einem Speicher abgelegt sein, der physisch im Mobiltelefon/Tablet aufgenommen ist und die Datenbank 20 befindet sich auf einem Cloud-Speicher, was einen einfachen Zugriff per PC zur Datenpflege und -eingabe ermöglicht. Die Invertierung ist gleichermaßen möglich. Auch kann die App als von einem Dienstleister bereitgestellte Servicefunktion für das Mobiltelefon/Tablet realisiert werden.

Der Schutzbereich ist nicht auf die erläuterten Ausführungsformen eingeschränkt. Verschiedene Modifikationen oder Alternativen sind für einen Fachmann innerhalb des Schutzbereichs möglich, welcher ausschließlich durch die Ansprüche definiert ist.

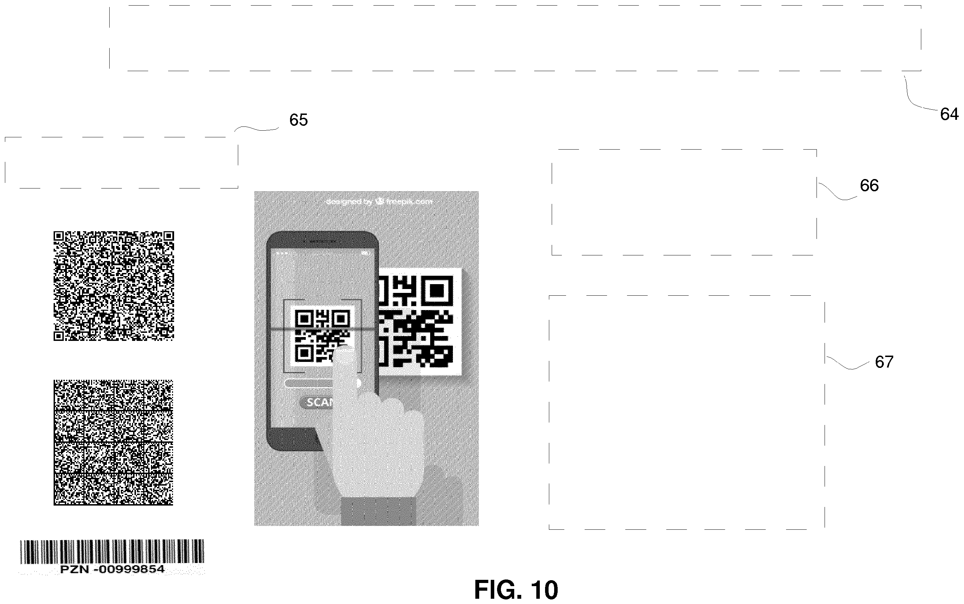

Die Bezugszeichen in den Figuren 7 bis 10 stehen für folgende Texte:
- 45: Patient zeigt Arzt seine Daten mit der App
- 46: Auswahl ärztlich relevanter Daten
- 47: App erstellt Barcode
- 48: Arzt importiert Daten per Barcode in sein IT-System
- 49: Arzt stellt Daten für den Patienten als Barcode bereit
- 50: Patient importiert Daten per Barcode in seine App
- 51: Patient erfasst seine Daten mit der App
- 52: Berichte früherer Arztbesuche/Behandlungen, z.B. Laborbefunde, Diagnosen, Arztbriefe, Hausarzt-/Facharztbesuche, Klinikaufenthalte
- 53: Symptome, z.B. Schmerz; Messungen, z.B. Blutdruck; Bisherige Medikation
- 54: z.B. Medikationsplan, Arztbrief, Laborwerte
- 55: Patient erfasst seine Daten mit der App
- 56: Symptome, z.B. Schmerz
- 57: Messungen, z.B. Blutdruck
- 58: Bisherige Medikation: Texteingabe oder Barcode
- 59: Berichte früherer Arztbesuche/Behandlungen, z.B. Laborbefunde, Diagnosen, Arztbriefe, Hausarzt-/Facharztbesuche, Klinikaufenthalte; Texteingabe oder Barcode
- 60: Migräneanfall
- 61: RR = 120/80
- 62: Automatischer Zeitstempel
- 63: Bereitstellen von ausgewählten Patientendaten als Barcode
- 64: Einscannen von Patientendaten
- 65: Verschiedene Barcode-Typen: z.B. QR-Code, Datamatrix, 1D-Barcode
- 66: Verschiedene Texte: z.B. Arztbrief, Medikationsplan oder einzelnes Medikament
- 67: Sehr geehrter Herr Kollege, wir berichten über den Patienten Werner Test, geb. 1.10.1960, der sich vom 1.10.2017 bis 10.10. 2017 in unserer stationären Behandlung befand. Diagnosen: Akuter Myokardinfarkt bei koronarer 3-Gefäßerkrankung ... Medikationsplan für Jürgen Wernersen 24.3.40 Praxis Dr. Michael Müller (Schloßstr. 22 10555 Berlin; 030-1234567; dr.mueller@kbv-net.de), Metoprolol (Metoprololsuccinat 1A 95mg Retard-Tabletten) 1-0-0 Stück Grund: Herz/Blutdruck ... Xylometazolin (Nasenspray Ratiopharm KDR PZN: 00999854)

In Weiterbildungen verfügt das Computerprogramm über ein Eingabemodul zur Eingabe von Medizindaten in die Datenbank über eine benutzerbediente Eingabeeinrichtung. Dies kann insbesondere eine Tastatur sein, die beispielsweise als Bildschirmtastatur auf einem Tablet ausgebildet ist. Über diese Eingabeeinrichtung kann der Benutzer vor dem Übertragen die Medizindaten in die Datenbank eingeben.

Diese Option ist besonders vorteilhaft für die Anamneseerhebung in einer Arztpraxis. Ein Patient erhält dann ein entsprechendes Gerät, beispielsweise ein Tablet, das eine noch unbefüllte Datenbank aufweist. Er gibt in diese Datenbank Medizindaten in Form von Anamneseangaben ein. Auf diese Weise wird die Datenbank mit Medizindaten befüllt. Anschließend wird vom Gerät, beispielsweise Tablet, die Übertragung per Barcode in das EDV-System des Arztes vorgenommen. Dazu werden die Medizindaten, wie in den Ausführungsbeispielen vorgesehen, entsprechend ausgewählt und in einem Barcode kodiert. Der Auswahlvorgang muss dabei nicht zwingend vom Patienten alleine oder selbst durchgeführt werden. Gleichermaßen ist es möglich, dass der Patient das Tablet o. ä. einer Praxiskraft übergibt, welche die Medizindaten auswählt. Dieser Auswahlschritt hat zum einen den Vorteil, dass nur diejenigen Medizindaten in den Barcode kodiert werden, die vom Arzt auch tatsächlich benötigt werden. Fehlerhafte Angaben können beispielsweise ausgeschlossen werden. Alternativ oder zusätzlich kann die Auswahl sich auch daran orientieren, ob die übertragene Datenmenge in einen Barcode passt. Das Tablett kann auf diese Weise als Art "elektronisches Klemmbrett" zur Patientenanamnese verwendet werden. Gleichermaßen kann es auch dazu dienen, dass bei Untersuchungen eine Praxiskraft Medizindaten in die Datenbank einträgt und an diese Medizindaten auswählt und in die elektronische Patientenakte des Arztes per Barcode überträgt.

So ist es in einer Weiterbildung vorgesehen, dass der Patient z. B .fünf Fragebögen ausführt und die einzelnen Fragebögen ausfüllt, um Medizindaten in die Datenbank einzutragen. Anschließend werden die Medizindaten der einzelnen fünf Fragebögen nacheinander ausgewählt, so dass insgesamt fünf Barcodes kodiert und übertragen werden. Wurde z. B. der vierte Fragebogen oder ein bestimmtes Feld falsch ausgefüllt, kann er/es bei der Auswahl unberücksichtigt bleiben und wird nicht in einem Barcode kodiert.

Weiter ist es optional vorgesehen, dass die Datenbank nach dem Übertragen der Medizindaten oder eine bestimmte Zeitspanne nach dem Eingeben oder Übertragen der Medizindaten automatisch so weit gelöscht wird, dass keine Medizindaten mehr enthalten sind. Dieser Löschvorgang kann auch mit der Eingabe eines bestimmten Datums, beispielsweise eines Patientennamens etc. automatisiert ausgelöst werden. Auf diese Weise kann ausgeschlossen werden, dass irrtümlich Medizindaten verschiedener Patienten gemischt werden.

In einer in dieser Ausführungsform oder auch grundsätzlich in Frage kommenden Weiterbildung wird der Tatsache Rechnung getragen, dass in ärztlichen EDV-Systemen die Verwendung von Freitextfeldern weit verbreitet ist. Dies hat zwar einerseits den Vorteil, dass beliebige Daten eingegeben werden können, andererseits den Nachteil, dass eine automatisierte Verarbeitung der Daten aus den Freitextfeldern mitunter schwierig ist. Dies kann bei der Eingabe der Medizindaten und insbesondere bei der Kodierung in den Barcode vorteilhaft korrigiert werden, indem das Kodierungsmodell einzelne Medizindaten durch Strukturierungszeichen in den Freitextfeldern voneinander trennt, die einerseits für einen Menschen lesbar sind, andererseits EDV-basiert die computergestützte Strukturierung der Daten ermöglicht. Ein Beispiel hierfür wäre:
"Größe: 187 cm; Gewicht: 90 kg;; Allergien: Latex;; Medikamente: ASS;; Treppensteigen: mit Mühe; Atemnot/Brustschmerz: nur bei Anstrengung;; Kardiovaskulär: hoher Blutdruck;; Pulmonal: nein;; Gastrointestinal: nein;; Neurologisch/Psychiatrisch: Depression;; Infektionskrankheit: nein;; Implantate: künstliche Hüfte links;; Glaukom: nein;; OP: Hüfte links vor drei Jahren;; Rauchen: ja, bis fünf Zigaretten/Tag;; Alkohol/Drogenabusus: 1 Bier;; Arztbrief an: Dr. Schmidt".

In dieser Aufgliederung steht jeweils vor einem Doppelpunkt ein Datenfeld und nach dem Doppelpunkt der Wert des Datenfelds. Der ";" trennt Datenfelder. Ein doppelter ";" trennt Datengruppen. Eine derartige Strukturierung ist rein beispielhaft, zeigt aber, dass sie für einen Benutzer leicht lesbar ist und dennoch eine EDV-gestützte Strukturierung ermöglicht; beispielsweise kann auf Basis dieser Trennung eine Formatierung des Freitextes zur besseren Anzeige erfolgen etc. Es ist deshalb bevorzugt, eine derartige Strukturierung in den Barcode aufzunehmen. Dies ist natürlich besonders vorteilhaft, wenn die Information von einem Benutzer in die Datenbank eingegeben wird und bei der Eingabe die für den Barcode verfügbaren Strukturierungszeichen berücksichtigt werden. Dann ist nicht nur eine Strukturierung möglich, sondern Medizindaten können gemäß der Strukturierung auch in mehrere Barcodes kodiert und so getrennt und/gruppiert ausgegeben werden. Das erschließt eine Anpassung der Auswahl und/oder Kodierung an verschiedene Arten von Zielsystemen, z.B. elektronische Patientenakten, Qualitätssicherungsdokumentationen, wissenschaftliche Studiendatenbanken.

Eine weitere Option ist es, in den Barcode eine digitale Signierung der eingegebenen Daten aufzunehmen. So kann beispielsweise die Information aus einem Arztbrief an die App mittels eines Barcodes übertragen werden, der eine digitale Signatur über Richtigkeit und Ersteller der Medizindaten enthält. Es ist bevorzugt, eine solche digitale Signatur bei der Weitergabe der Medizindaten ebenfalls zu übertragen. Auf diese Weise wird beispielsweise von einem ersten Arzt ein erster Befund umfassend Medizindaten an die Datenbank übertragen, wobei im Barcode eine digitale Signatur über die Richtigkeit und die Ausstellung durch den ersten Arzt enthalten ist. Diese Signatur wird in der Datenbank zu den Medizindaten passend gespeichert und bei der Übertragung der Medizindaten an einen zweiten Arzt in den Barcode einkodiert. Dadurch hat der zweite Arzt Kenntnis über Zuverlässigkeit und Quelle der Medizindaten. Die Signatur kann beispielsweise als Zahlencode erstellt werden. Mittel zum Signieren von Daten bezüglich Vollständigkeit und Quelle sind dem Fachmann bekannt, beispielsweise in Form des sogenannten Hash-Codes.

## Patentansprüche

1. Computerprogramm zum Verwalten von auf einen Patienten bezogenen Medizindaten, wobei das Computerprogramm (16) Programmcode aufweist, der zur Ausführung auf einem Prozessor (4) eines Mobiltelefons (2) oder Tablets ausgebildet ist, das einen Bildschirm (8), eine Eingabevorrichtung (10), und einen Datenspeicher (16) aufweist, **dadurch gekennzeichnet, dass** das Computerprogramm (16) aufweist:
- ein Datenbankmodul (24), das in einer Datenbank (20) die Medizindaten (22) verwaltet,
- ein Auswahlmodul (26), das auf Anforderung durch einen Benutzer aus der Datenbank (20) mehrere der Medizindaten (22) ausliest und auf dem Bildschirm (8) eine Auswahlanzeige darstellt, welche die mehreren der Medizindaten (22) zur Auswahl anzeigt, und die angezeigten Medizindaten (22) über die Eingabevorrichtung (10) als ausgewählt markierbar macht,
- ein Kodierungsmodul (28), das nur markierte und damit ausgewählte Medizindaten (22) in einen Barcode (36) kodiert und
- ein Übertragungsmodul (30), das den Barcode (36) auf einem Medium zum Einlesen durch einen Gesundheitsdienstleister bereitstellt.

2. Computerprogramm nach Anspruch 1, **dadurch gekennzeichnet, dass** das Mobiltelefon (2) oder Tablet eine Barcode-Leseeinrichtung (12) aufweist und das Computerprogramm (16) ein Eingabemodul (32) aufweist, das einen Barcode (36) mittels der Barcode-Leseeinrichtung (12) einliest und zur Dekodierung der Medizindaten (22) aus dem Barcode (36) an das Kodiermodul (28) übergibt.

3. Computerprogramm nach Anspruch 1 oder 2, **gekennzeichnet durch** ein Eingabemodul zur Eingabe von Medizindaten (22) in die Datenbank (20) über eine benutzerbediente Eingabeeinrichtung, insbesondere eine Tastatur, z. B. eine Bildschirmtastatur.

4. Computerprogramm nach einem der Ansprüche 1 bis 3, **dadurch gekennzeichnet, dass** das Kodierungsmodul (28) die ausgewählten Medizindaten (22) zum Einlesen in ein Freitextfeld aufbereitet und dabei die Medizindaten (22) durch benutzer- und computerlesbare Strukturierungszeichen strukturiert, und/oder das Kodierungsmodul (28) für die Medizindaten (22) eine digitale Signaturinformation in den Barcode (36) aufnimmt.

5. Vorrichtung zum Verwalten von auf einen Patienten bezogenen Medizindaten (22), die als Mobiltelefon (2) oder Tablet ausgebildet ist, das einen Bildschirm (8), eine Eingabevorrichtung (10), und einen Datenspeicher (16) aufweist, wobei die Vorrichtung (2) ein Computerprogramm (16) nach einem der Ansprüche 1 bis 4 aufweist.

6. System zum Verwalten von auf einen Patienten bezogene Medizindaten (22) umfassend eine Vorrichtung (2) nach Anspruch 5 sowie mind. eine Datenbank (38) für Gesundheitsdienstleister zur Verwaltung von Medizindaten (22) mehrerer Patienten und eine Barcode-Lese- und Dekodiervorrichtung (40) zur Eingabe von in einem Barcode (36) kodierten, auf einen bestimmten Patienten bezogenen Medizindaten (22).

7. System nach Anspruch 6, **gekennzeichnet durch** Medizindatenkodier- und -ausgabeeinrichtung (42) zur Kodierung vom Gesundheitsdienstleister ausgewählter, auf den Patienten bezogene Medizindaten (22) in einen Barcode (36) und dessen Ausgabe auf einem Medium.

8. Verfahren zum Verwalten von auf einen Patienten bezogenen Medizindaten (22), für die ein vom Patienten zu verwendender Datenspeicher (6) vorgesehen wird, wobei
- im Datenspeicher (6) eine Datenbank (20) für die Medizindaten (22) vorgesehen ist,
- die Medizindaten (22) einem Gesundheitsdienstleister zur Verfügung gestellt werden, wobei ein Barcode (36) Verwendung findet,
**dadurch gekennzeichnet, dass**
- der Datenspeicher (6) in ein Mobiltelefon (2) oder Tablet integriert ist, das einen Bildschirm (8) und eine Eingabevorrichtung (10) aufweist, und
- die Medizindaten (22) an den Gesundheitsdienstleister übertragen werden, indem mittels Bildschirm (8) und Eingabevorrichtung (10) in der Datenbank (20) zu übertragende Medizindaten (22) ausgewählt werden, das Mobiltelefon (2) oder Tablet nur die ausgewählten Medizindaten (22) in den Barcode (36) kodiert und diesen auf einem Medium zum Einlesen bereitstellt.

9. Verfahren nach Anspruch 8, **dadurch gekennzeichnet, dass** der Patient vor dem Übertragen die Medizindaten (22) in die Datenbank (20) über die Eingabevorrichtung eingibt.

10. Verfahren nach Anspruch 8 oder 9, **dadurch gekennzeichnet, dass** die ausgewählten Medizindaten (22) zum Einlesen in ein Freitextfeld aufbereitet und dabei die Medizindaten (22) durch benutzer- und computerlesbare Strukturierungszeichen strukturiert werden, und/oder das Kodierungsmodul (28) für die Medizindaten (22) eine digitale Signaturinformation in den Barcode (36) aufnimmt.

11. Verfahren nach einem der Ansprüche 6 bis 10, **dadurch gekennzeichnet, dass** das Mobiltelefon (2) oder Tablet eine Barcode-Leseeinrichtung (12) aufweist und bei einem Gesundheitsdienstleister erzeugte Medizindaten (22) im Datenspeicher (6) ablegt, indem der Gesundheitsdienstleister die Medizindaten (22) in einen Barcode (36) kodiert, diesen bereitstellt, der Patient den Barcode (36) mittels der Barcode-Leseeinrichtung (12) einliest und das Mobiltelefon (2) oder Tablet die Medizindaten (22) aus dem Barcode (36) dekodiert und in der Datenbank (20) ablegt.

12. Verfahren nach Anspruch 11, **dadurch gekennzeichnet, dass** der Gesundheitsdienstleiser den Barcode (36) ausdruckt.

13. Computerprogramm, Vorrichtung, System oder Verfahren nach einem der obigen Ansprüche, **dadurch gekennzeichnet, dass** das Medium der Bildschirm (8) des Mobiltelefons (2) oder Tablets ist.

14. Computerprogramm, Vorrichtung, System oder Verfahren nach einem der obigen Ansprüche, **dadurch gekennzeichnet, dass** bei der Kodierung und/oder Dekodierung des Barcodes (36) nur druckbare Zeichen oder Zeilenschaltungen berücksichtigt werden.

15. Computerprogramm, Vorrichtung, System oder Verfahren nach einem der obigen Ansprüche, **dadurch gekennzeichnet, dass** für den Barcode (36) eine maximale Speicherdichte vorgegeben ist und bei Überschreiten dieser Speicherdichte zur Kodierung eine Abfolge mehrerer Barcode-Elemente erzeugt wird.
